Europäisches Patentamt

European Patent Office

Office européen des brevets

(11) Veröffentlichungsnummer: **0 380 672**
**A1**

(12) **EUROPÄISCHE PATENTANMELDUNG**
veröffentlicht nach Art. 158 Abs. 3
EPÜ

(21) Anmeldenummer: 89900366.9

(22) Anmeldetag: 29.07.88

(86) Internationale Anmeldenummer:
PCT/SU88/00156

(87) Internationale Veröffentlichungsnummer:
WO 90/01493 (22.02.90 90/05)

(51) Int. Cl.5: **C07K 1/04**

(43) Veröffentlichungstag der Anmeldung:
08.08.90 Patentblatt 90/32

(84) Benannte Vertragsstaaten:
**CH DE FR GB IT LI SE**

(71) Anmelder: **SPETSIALNOE KONSTRUKTORSKOE BJURO BIOLOGICHESKOGO PRIBOROSTROENIA AKADEMII NAUK SSSR**
Moskovskaya obl.
puschino, 142292(SU)

(72) Erfinder: **BARU, Mikhail Borisovich**
mikroraion AB, 23-10 Moskovskaya obl.
Puschino, 142292(SU)
Erfinder: **RODIONOV, Igor Leonidovich**
mikroraion D, 1-161 Moskovskaya obl.
Puschino, 142292(SU)
Erfinder: **SHESTAKOVSKY, Leonid Yakovlevich**
mikroraion G, 9-46
Moskovskaya obl. Puschino, 142292(SU)
Erfinder: **LARIN, Vyacheslav Tarasovich**
mikroraion AB, 24-20 Moskovskaya obl.
Puschino, 142292(SU)

(74) Vertreter: **von Füner, Alexander, Dr. et al**
**Patentanwälte v. Füner, Ebbinghaus, Finck**
**Mariahilfplatz 2 & 3 Postfach 95 01 60**
**D-8000 München 95(DE)**

(54) **VERFAHREN ZUR REGELUNG DER FESTPHASEN-SYNTHESE VON BIOPOLYMEREN.**

(57) Die Kontrolle der an einem schlaffen polymeren Träger durchführbaren Festkörpersynthese von Biopolymeren erfolgt durch Bestimmung des Initialvolumens des Trägers und anschließende Messung des Volumens des Trägers mit einem daran gebildeten Stoff im Laufe jeder Synthesestufe. Man ermittelt die summarische Volumenzunahme des Trägers mit dem daran gebildeten Stoff und, falls die Zunahme gleich Null ist, verwirklicht die darauffolgende Synthesestufe.

EPAA-38917.3

# VERFAHREN ZUR KONTROLLE DER FESTKÖRPERSYNTHESE VON BIOPOLYMEREN

## Gebiet der Technik

Die Erfindung bezieht sich auf die Festkörpersynthese von Biopolymeren und insbesondere auf ein Verfahren zur Kontrolle der Festkörpersynthese von Biopolymeren.

## Zugrundeliegender Stand der Technik

Die Festkörpersynthese von Biopolymeren besteht darin, daß an einem polymeren Träger die Kette eines Biopolymers (bestehend zum Beispiel aus einer Reihe von Aminosäuren) Schritt für Schritt wächst.

Der Vorteil der Festkörpersynthese von Biopolymeren gegenüber Synthese derselben in Lösungsmitteln besteht in der Sicherstellung einer leichteren Trennung des herstellbaren Endprodukts, der in Überschuß genommenen Reagenzien und gelösten Nebenprodukten.

Die Synthese von Biopolymeren wie ein beliebiger Syntheseprozeß ist ohne Kontrolle des vollständigen Verlaufs jeder Synthesestufe nicht möglich. Die Sicherheit, daß die eine Synthesestufe vollendet worden ist, gibt die Möglichkeit, mit der Durchführung der darauffolgenden Synthesestufe zu beginnen. Die Kontrolle des Syntheseprozesses ist gewöhnlich mit großen Schwierigkeiten verbunden und arbeitsaufwendig, weil sie mit Hilfe der Dünnschicht- oder Hochleistungschromatografie erfolgt.

Heute ist bekannt ein Verfahren zur Kontrolle der Festkörpersynthese von Biopolymeren mittels Änderung der physikalisch-chemischen Eigenschaften eines Trägers, zum Beispiel der Granalien von Styrol-Divinylbenzol-Mischpolymerisat, gebunden an im Laufe der Synthese gewonnenes Peptid, wobei dieses Verfahren in der Bestimmung der Differenz zwischen Masse dieses Trägers mit dem bei der gegebenen Anzahl der Kondensationen von Aminosäuren gewonnenen Peptid und Initialmasse des Polymers besteht. Die Massezunahme des polymeren Trägers kann eine annähernde Menge des am Polymer erhaltenen Peptids anzeigen. Zur Ermittlung der genannten Differenz wird eine gewisse Menge des Trägers mit dem daran gebildeten Peptid aus dem Reaktor, worin der Syntheseprozeß durchgeführt wird, isoliert, dann getrocknet und gewogen.

Die für die Durchführung der genannten Arbeitsschritte erforderliche Zeit ist auf das Mehrfache größer als die Dauer jeder Synthesestufe. Dieses Verfahren zur Synthesekontrolle macht es außerdem erforderlich den Syntheseprozeß zu unterbrechen (Dzh. Stjuart, Dzh. Yang. Tverdofazny sintez pepridov, 1971, Verlag "Mir" (Moskau), S. 88).

Bei den anderen bekannten Verfahren zur Synthesekontrolle, welche auf der Isolierung einer gewissen Menge des Trägers mit dem Peptid oder auf der Ausnutzung des ganzen Trägervolumens beruhen, ist es ebenfalls erforderlich, den Syntheseprozeß abzubrechen. Diese Verfahren sind mit dem Nachweis der Hauptaminogruppen am Träger durch Behandlung des letztgenannten mit überschüssigem Anion, welches leicht zu bestimmen ist, verbunden (Reaktsii na polimernykh podlozhkakh v organicheskom sinteze, 1983, Verlag "Mir" (Moskau), S. 454-455).

Neben Syntheseabbruch bzw. Verlängerung der Synthesezeit ergeben sich dabei folgende Nachteile, nämlich Möglichkeit, Peptid oder sein Fragment zu schädigen, Trägerverluste, verbunden mit dem Isolieren des Trägers aus der Reaktionsmasse, unzureichende Kontrollgenauigkeit, Kompliziertheit der Automatisierung und Steuerung des Syntheseprozesses. Die Unvollkommenheit der bekannten Verfahren zur Synthesekontrolle, welche keine Angaben über die Beendigung jeder Synthesestufe liefern, führt dabei zum Überverbrauch an Lösungsmitteln und Reagenzien (2- bis 4facher Überschuß).

Zur Verwirklichung einer zuverlässigeren Kontrolle der Kondensation von Aminosäuren bei der Festkörpersynthese von Peptiden wird die Ninhydrinreaktion genutzt. Dazu wird ein Trägerteil dem Reaktor entnommen und nach mehrmaligem Waschen mit Reagenzien behandelt, wodurch der Träger eine bestimmte Farbe erhält, welche die Tiefe der Kondensationsreaktion angibt (Reaktsii na polimernykh podlozhkakh v organicheskom sinteze, 1983, Verlag "Mir" (Moskau), S. 455-456).

Aber auch für dieses Verfahren sind Verluste an Träger, Reagenzien und Endprodukt, Möglichkeit, Peptid zu schädigen, und Verlängerung der Synthesezeit charakteristisch.

Bekannt ist ferner ein Verfahren zur Kontrolle der Festkörpersynthese, das darin besteht, daß man ins Reaktionsvolumen nach der Beendigung der Kondensationsstufe die Pikrinsäure einführt, mit deren Hilfe die Vollständigkeit der Synthesereaktion von Peptiden bestimmt wird d.h. freie nichtumgesetzte Aminogruppen schnell nachgewiesen werden.

Auch diese Kontrollmethodik ruft die Schädigung von Peptid entweder durch teilweise Spaltung von gegen Wirkung der Pikrinsäure unbeständigen Aminogruppen oder durch Schädigung der anderen empfindlichen Fragmente hervor. Da die Kontrolle des Syntheseprozesses unter Nutzung des ganzen Reaktionsvolumens geführt wird, so stören die ausgelösten oben angegebenen Nebenreaktionen die Gesamtsynthese von Biopolymeren (Reaktsii na polimernykh podlozhkakh v organicheskom sinteze, 1983, Verlag "Mir" (Moskau), S. 455).

In Gebrauch sind ebenfalls die optischen Verfahren zur Kontrolle der Festkörpersynthese von Biopolymeren, die auf der UV-Lichtabsorption basieren. Das Auftreten der betreffenden Bande bei Reaktionslösungen durch Entblockung der Schutzgruppen und das Verschwinden dieser Bande an einem Spektralphotometer im Falle der Kondensation kann als Zeichen für den Reaktionszustand dienen. Diese Kontrolle sichert jedoch keine Möglichkeit, die Reaktionsbeendigung zu ermitteln, und gestattet nicht, die Angaben über den echtzeitlichen Reaktionsverlauf zu gewinnen (Reaktsii na polimernykh podlozhkakh v organicheskom sinteze, 1983, Verlag "Mir" (Moskau), S.454).

Alle bekannten chemischen und spektralphotometrischen Verfahren zur Kontrolle der Reaktionen und Stufen der Festkörpersynthese von Biopolymeren machen es unmöglich, die kontinuierliche Kontrolle des Syntheseprozesses durchzuführen, bei der Synthese von Biopolymeren benutzt man deshalb sehr große Mengen von teuren Spülflüssigkeiten und Reagenzien, die Durchführung jeder Synthesestufe wird bedeutend verlängert, mehrmals werden die einzelnen Arbeitsschritte wiederholt, verwendet werden teure Durchflußspektralphotometer (Dzh. Stjuart, Dzh. Yang. Tverdofazny sintez peptidov, 1971, Verlag "Mir" (Moskau), S. 82-93).

Es gibt eine hohe Schädigungswahrscheinlichkeit bei

einem synthetisierbaren Biopolymer oder selbst der vollständige Verlust an Biopolymer wegen Störung seiner chemischen Zusammensetzung.

Offenbarung der Erfindung

Der vorliegenden Erfindung liegt die Aufgabe zugrunde, durch Ausnutzung der Abhängigkeit der Volumenänderung eines Trägers vom Reaktionsverlauf bei der Festkörpersynthese von Biopolymeren ein Verfahren zur Kontrolle der Festkörpersynthese von Biopolymeren zu entwickeln, welches ermöglicht, die zerstörungsfreie Prüfung des Endprodukts während seiner Synthese vorzunehmen, die Synthesedauer zu verkürzen, die Mengen von an der Synthese beteiligten Stoffen zu vermindern.

Diese Aufgabe wird dadurch gelöst, daß man in einem Verfahren zur Kontrolle der an einem schlaffen polymeren Träger durchführbaren Festkörpersynthese von Biopolymeren erfindungsgemäß das initiale Schüttvolumen des Trägers bestimmt, im Laufe jeder Synthesestufe das Volumen des Trägers mit einem auf dieser Synthesestufe gebildeten Stoff mißt, die summarische Volumenzunahme des Trägers mit dem daran gebildeten Stoff ermittelt und, falls die Volumenzunahme erreicht wird, die gleich Null ist, die darauffolgende Synthesestufe verwirklicht.

Dank der vorliegenden Erfindung wird die Gesamtzeit der Synthese um 50 % und mehr verringert, die Güte des hergestellten Produkts ist bedeutend höher, weil verschiedene mit den bekannten Kontrollverfahren einhergehende Störungen ausgeschlossen werden. Die Menge von Reagenzien, welche bei der Synthese von Biopolymeren verbraucht werden, wird auf ein Viertel bis ein Achtel verringert.

Gemäß der vorliegenden Erfindung ist es zweckmäßig, die Durchführung der darauffolgenden Synthesestufe, falls die Volumenzunahme des Trägers, die gleich Null ist, erreicht wird, um die Zeit aufzuhalten, die für die völlige Beendigung der vorhergehenden Synthesestufe erforderlich ist, was es möglich macht, die Anzahl der Arbeitsschritte bei der Kondensation der einen Aminosäure auf das 2,4fache zu verringern, und die Menge von Lösungsmitteln, welche je 1 Kondensation verbraucht werden, auf ein Viertel bis ein Achtel zu vermindern.

Weitere Ziele und Vorteile der erfindungsgemäßen Erfindung sind aus der nachfolgenden ausführlichen Beschreibung des Verfahrens zur Kontrolle der Festkörpersynthese von Biopolymeren und der konkreten Ausführungsbeispiele ersichtlich.

Kurze Beschreibung der Zeichnung

Die Zeichnung zeigt die zeitliche Volumenänderung des polymeren Trägers während der Festkörpersynthese von Biopolymeren.

Beste Ausführungsvariante der Erfindung

Das erfindungsgemäße Verfahren zur Kontrolle der Festkörpersynthese von Biopolymeren, zum Beispiel der Synthese von Peptiden, Polypeptiden, Nulkeotiden, Oligonukleotiden, Hormonen, Alkaloiden, Insektenlockstoffen, Karotinoiden, Desoxysacchariden, Oligosacchariden beruht auf der Fähigkeit eines polymeren Trägers, sein Volumen um das 1,05- bis 9,0fache und darüber im Laufe der Syntheseopreationen zu vergrößern.

Die bei der Festkörpersynthese von Biopolymeren verwendeten schlaffen polymeren Träger stellen Granalien aus vernetzten Polymeren zum Beispiel aus Styrol-Divinylbenzol-Mischpolymerisat, Akrylamid, Diakrylamid, vernetzten hydrofoben Dextranen dar.

Zur Verwirklichung des erfindungsgemäßen Verfahrens läßt sich ein Reaktor einsetzen, dessen Aufbau den ständigen Kontakt von Granalien des Trägers miteinander sichert.

Es ist festgestellt, daß jede der Synthesestufen (Blokkung, Entblockung, Waschen, Kondensation) zur Volumenänderung der Reaktionsmasse (Volumenvergrößerung oder -verminderung) führt. Diese Volumenänderung hängt von der numerischen Reihenfolge der Stufe, der chemischen Zusammensetzung der wachsenden Biopolymerenkette und der Art des Trägerpolymers ab. Es ist gefunden, daß die Volumenänderung des Trägers unter den oben angegebenen Bedingungen konstant ist, und daß jede Synthesestufe des Biopolymers dann völlig beendigt ist, wenn die weitere Volumenänderung (Volumenvergrößerung oder -verminderung) der Reaktionsmasse nicht stattfindet.

Bei der Synthese der Valin-Glyzin-Peptidbindung erhöht sich zum Beispiel das Volumen des polymeren Trägers auf der Stufe der Kondensation von tert. Butyloxykarbonylglyzin um 37 konventionelle Volumeneinheiten gegenüber an Träger früher gebundenem tert. Butyloxykarbonylvalin. Die Unterbrechung der Volumenänderung (die Volumenzunahme ist gleich Null) während der Synthese wird durch vollständigen Anbau von Reagenzien an freie Bindungen und Beendigung des Eindringens von Lösungsmitteln in alle Poren des Trägerpolymers erreicht. Je nach den physikalisch-chemischen Eigenschaften des Trägers, welche durch seine Natur, numerische Reihenfolge von Syntheseoperationen und -stufen, chemische Zusammensetzung der wachsenden Biopolymerenkette gekennzeichnet wird, verändert sich das Volumen des Trägers, wie hingewiesen wurde, um das 1,05 bis 9,0fache und darüber.

Das erfindungsgemäße Verfahren zur Kontrolle der Festkörpersynthese von Biopolymeren besteht darin, daß das initiale Schüttvolumen eines Trägers, an dem die Synthese durchgeführt wird, bestimmt und nach der Einführung von Reaktionsstoffen in den Reaktionsraum das Schüttvolumen des Trägers mit einem daran gebildeten Stoff gemessen wird. Falls die Volumenzunahme des Trägers Null erreicht, ist es vorteilhaft, die darauffolgende Synthesestufe nach gewissem Halten der Reaktionsmasse unter Bedingungen der vorhergehenden Synthesestufe zu beginnen. Die empfohlene Haltedauer (1 bis 10 Minuten) läßt der vorhergehenden Synthesestufe dann vollständig zum Abschluß kommen, wenn die Stufe zu 98 bis 99 % praktisch vollendet ist und die weitere Volumenänderung des Trägers so geringfügig ist, daß sie praktisch nicht nachgewiesen werden kann.

Nach der völligen Beendigung der einen Synthesestufe, die durch Volumenzunahme des Trägers, welche gleich Null ist, gekennzeichnet wird, und möglicherweise nach gewissem Halten der Reaktionsmasse kann man zur Verwirklichung der darauffolgenden Synthesestufe gehen, deren Verlauf ständig überacht wird, wobei die Volumenänderungen des Trägers festgelegt werden.

Die Volumenzunahme des Trägers auf jeder Synthesestufe einmal bestimmt kann man die Masse des am Träger kondensierten Stoffs mit einem geringen Fehler ermitteln und durch Vergleich derselben mit der berechneten Masse die vollständige Reaktion in Zahlen (Prozent) bestimmen..

Ausgehend von der Volumenzunahme des Trägers mit daran gebundenen Kettenbausteinen des Biopolymers kann die Kontrolle und Steuerung des Syntheseprozesses durchgeführt werden. Durch Einsatz eines Rechners bei der Synthesesteuerung mittels Volumenzunahme kann man zu beliebiger Synthesezeit die Information über die Menge und Güte des wachsenden Biopolymers zur Verfügung haben und die Synthesebedingungen im Laufe des Syntheseprozesses automatisch ändern, indem man bessere Ergebnisse gewinnt.

Das erfindungsgemäße Verfahren zur Kontrolle der Festkörpersynthese von Biopolymeren macht es möglich (gegenüber dem in US, A, 4382922 angemeldeten Verfahren), den Verbrauch an teuren und zu wenig vorhandenen Reagenzien auf das Mehrfache durch rechtzeitige Vollendung jeder Synthesestufe nach einem Signal, das über die Aufhebung der Volumenänderung des Trägers informiert, herabzusetzen.

Beispiel 1

In einem kontinuierlichen Reaktor, der mit einem granulierten schlaffen polymeren Träger und zwar Styrol-Divinylbenzol-Mischpolymerisat eingefüllt ist, wird ein Peptid (ß-Kasomorphinanalogon) Tyr-Pro-Phe-NH$_2$ synthetisiert. Die Temperatur von Reagenzien und Lösungsmitteln beträgt $20 \pm 3^{\circ}C$, der Druck ist atmosphärisch.

Vor Synthesebeginn führt man in den Reaktor Dimethylformamid und dann Methanol zum Waschen des Trägers ein, indem man die Quellbarkeit des Trägers prüft.

Man registriert die Größe des Initialvolumens des Trägers.

Man führt in Dimethylformamid genommenes tert. Butyloxykarbonylvalin in den Reaktor ein, welches am Träger kondensiert. Das Volumen des Trägers mit dem daran kondensierten Stoff wird als Nullwert der Volumenzunahme angenommen.

Dem Reaktor wird Methylenchlorid zum Waschen des Trägers mit dem daran kondensierten Stoff zugeführt. Die wachsende Volumenzunahme gegenüber Nullwert wird registriert.
Falls die Volumenzunahme gleich Null ist, wird die Einführung von Methylenchlorid in den Reaktor abgestoppt. Innerhalb von 5 Minuten werden keine Verbindungen in den Reaktor
zugegeben, damit die Kondensation von Butyloxykarbonylvalin
am Träger völlig zum Abschluß kommt. Dann wird dem Reaktor
die 50%ige Lösung der Trifluoressigsäure in Methylenchlorid
zugeführt. Ununterbrochen wird die wachsende Volumenzunahme
des Trägers registriert. Falls die Volumenzunahme des Trägers
gleich Null ist, wird die Zufuhr der Trifluoressigsäure eingestellt und die Reaktionsmasse im Reaktor innerhalb von 6
Minuten gehalten. Dann führt man in den Reaktor Äthyldiisopropylamin, genommen in Methylenchlorid ein. Man registriert
das Anwachsen der Volumenzunahme des Trägers. Falls man feststellt, daß die Volumenzunahme des Trägers gleich Null ist,
hält die Reaktionsmasse im Reaktor innerhalb von 5 Minuten,
ohne daß man in den Reaktor Reagenzien zur vollständigen
Beendigung der Kondensationsreaktion am Träger einführt.
Dann wird dem Reaktor Methanol zum Waschen des Trägers zugeführt, wobei seine Volumenzunahme registriert wird. Wenn
die Volumenzunahme des Trägers den Nullwert erreicht, wird
die Zufuhr irgendwelcher Reagenzien in den Reaktor innerhalb
von 3 Minuten eingestellt. Dann führt man in den Reaktor
tert. Butyloxykarbonylglyzin, genommen in Dimethylformamid,
ein. Gleichzeitig wird die Volumenzunahme des Trägers registriert. Erreicht die Volumenzunahme des Trägers den Nullwert, so wird die Zufuhr von tert. Butyloxykarbonylglyzin
eingestellt. Die Reaktionsmasse hält man innerhalb von 7 Minuten, wonach man dem Reaktor Dimethylformamid zuführt und
die Volumenzunahme des Trägers registriert.

Das weitere Wachsen der Biopolymerenketten am Träger
erfolgt analog zu dem oben angegebenen, wobei die Volumenänderung des Trägers ständig registriert wird.

Nach der Totalsynthese des β-Kasomorphinanalogons am
Träger wird die Standardprüfung mit Pikrinsäure zwecks Kontrolle der vollständigen Synthese durchgeführt.

Die Massezunahme des Biopolymers beträgt 99,1 % der
theoretisch berechneten Größe. Das vom Träger abgenommene Biopolymer wird auf die chromatografische Homogenität geprüft. Die Reinheit beträgt 96 %.

Beispiel 2

In einem kontinuierlichen Reaktor, der mit einem schlaffen polymeren Träger und zwar Styrol-Divinylbenzol-Mischpolymerisat (mit 1,0 Gew.% Divinylbenzol) eingefüllt ist, wird die Val-Gly-Peptidkette aufgewachsen.

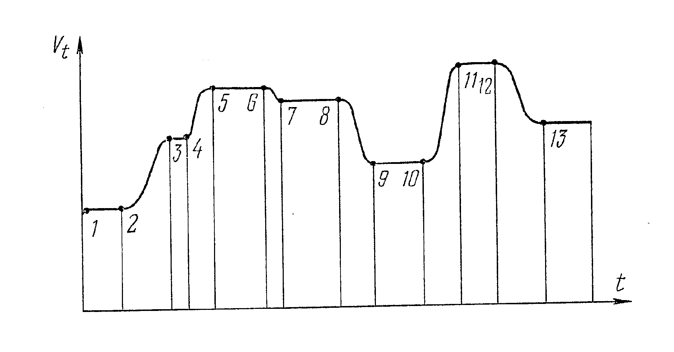

Die Volumenänderung des Trägers wird mit Hilfe eines Gebers aufgezeichnet, dessen Signale auf einen Selbstschreiber (in Fig. nicht gezeigt) übertragen werden, welcher die Volumenänderung des Trägers (Abszissenachse) als Zeitfunktion (Ordinatenachse) während der Synthese von Dipeptid grafisch darstellt. (Fig. 1).

Reagenzien und Lösungsmittel werden in den Reaktor durch eine Minipumpe gefördert, die Durchflußgeschwindigkeit beträgt 1,2 und 4 ml/min. Der Syntheseprozeß setzt beim Durchpumpen von Dimethylformamid durch den Reaktor zwecks Herstellung der Basislinie ein, von welcher die Abmessung der Volumenzunahme des Trägers erfolgen wird. Dann wäscht man das Polymer mit Methanol, indem man seine Quellbarkeit prüft. Dem Reaktor führt man tert. Butyloxykarbonylvalin, genommen in Dimethylformamid, zu, welches am Träger kondensiert. Das Volumen des Trägers mit dem daran kondensierten Stoff wird als Nullwert der Volumenzunahme angenommen (Position 1).

Der Träger mit dem gebildeten Peptid wird mit Methylenchlorid gewaschen. Die Volumenzunahme in konventionellen Einheiten (gegenüber Nullwert) beträgt 23,5 (Abschnitt 2-3). Falls die Volumenzunahme gleich Null ist (Position 3), wird die Zufuhr von Methylenchlorid eingestellt und die Durchführung der darauffolgenden Synthesestufe um 5 Minuten (Abschnitt 3-4) aufgehalten. Die Entblockungsstufe erfolgt durch Einführung der 50%igen Trifluoressigsäure in Methylenchlorid (Abschnitt 4-5) in den Reaktor, wobei die Volumenzunahme des Trägers 32 konventionelle Einheiten beträgt.

Falls die Volumenzunahme des Trägers gleich Null ist (Position 5), wird die 50%ige Trifluoressigsäure in Methylenchlorid weiter eingeführt, während die Durchführung der darauffolgenden Synthesestufe um 15 Minuten (Abschnitt 5-6) aufgehalten wird. Zwecks Neutralisation des Trägers mit Peptid führt man in den Reaktor Äthyldiisopropylamin in Methylenchlorid (Abschnitt 6-7) ein, die Volumenzunahme beträgt dabei 27 konventionelle Einheiten. Die Verwirklichung der darauffolgenden Synthesestufe wird um 15 Minuten (Abschnitt 7-8) aufgehalten. Danach führt man Methanol zum Waschen des erhaltenen Peptids (Abschnitt 8-9) in den Reaktor ein, fixiert die Volumenzunahme des Trägers, welche 11 konventionelle Einheiten beträgt. Die Durchführung der darauffolgenden Synthesestufe wird um 15 Minuten (Abschnitt 9-10) aufgehalten. Nach 15 Minuten vollzieht sich die Kondensation von tert. Butyloxykarbonylglyzin, das in den Reaktor als Lösung in Dimethylformamid eingeführt wird, wobei festgestellt wird, daß die Volumenzunahme 37 konventionelle Einheiten (Abschnitt 10-11) beträgt. Die Durchführung der darauffolgenden Synthesestufe wird dann um 10 Minuten (Abschnitt 11-12) aufgehalten. Hiernach führt man dem Reaktor Dimethylformamid zum Waschen des Trägers mit dem erhaltenen Stoff (Abschnitt 12-13) zu, es wird festgesetzt, daß die Volumenzunahme des Trägers 21 konventionelle Einheiten beträgt. Auf solche Weise ist Valin-Glyzin-Dipeptid erhalten.

Zur Kontrolle der vollständigen Synthese von Dipeptid wird die Standardprüfung mit Pikrinsäure vorgenommen.

Die Standardprüfung hat ergeben, daß der Peptidzuwachs 99% der theoretisch berechneten Größe beträgt.

Im Zusammenhang damit, daß das erfindungsgemäße Verfahren die kontinuierliche Kontrolle des Syntheseprozesses vorsieht, unterscheidet sich die Synthesemethodik von den bestehenden Standardmethoden wesentlich, was seinerseits unmöglich macht, den formalen Vergleich aller Syntheseoperationen durchzuführen. Nut einzelne Operationen können verglichen werden. Dieser Vergleich ist in Tabelle zu erkennen.

Tabelle

| Vergleichskriterien | Das erfindungsgemäße Verfahren | Das bekannte Verfahren (US,A, 4382922) |
| --- | --- | --- |

1. Anzahl von Operationen je
   1 Kondensation                  11          25

2. Menge von verwendeten Lösungsmitteln je 1 Kondensation in $cm^3$ 334       1620

3. Zeit, welche die Operationen
   der einen Kondensation in Anspruch nehmen, in Minuten      176 bis 196[x] 121 bis 391[xx]

[x] Angegeben ist die volle Zeit für alle Operationen, einschließlich der Kontrolle der Vollendung der Kondensationsreaktion.

[xx] Angegeben ist die reine Zeit des Vorliegens von Reagenzien und Lösungsmitteln im Reaktor. Die Zeit für die Einfüllung des Reaktors und den Reaktorabfluß, die die Zeit von Syntheseoperationen bedeutend übersteigt, ist nicht angeführt. Nicht berücksichtigt ist ebenfalls die Zeit, die für die Kontrolle der Vollendung der Kondensationsreaktion erforderlich ist, wobei sie zwischen 90 und 120 Minuten liegt.

EP 0 380 672 A1

## PATENTANSPRÜCHE

1. Verfahren zur Kontrolle der an einem schlaffen polymeren Träger durchführbaren Festkörpersynthese von Biopolymeren, d a d u r c h   g e k e n n z e i c h n e t, daß man das initiale Volumen des Trägers bestimmt, im Laufe jeder Synthesestufe das Volumen des Trägers mit einem auf dieser Synthesestufe gebildeten Stoff mißt, die summarische Volumenzunahme des Trägers mit dem daran gebildeten Stoff ermittelt und, falls die Volumenzunahme erreicht, die gleich Null ist, die darauffolgende Synthesestufe verwirklicht.

2. Verfahren nach Anspruch 1, d a d u r c h   g e - k e n z e i c h n e t, daß beim Erreichen der Volumenzunahme des Trägers, die gleich Null ist, die Durchführung der darauffolgenden Synthesestufe um die für die vollständige Beendigung der vorhergehenden Synthesestufe erforderliche Zeit aufgehalten wird.

EPAA-38917.3

# INTERNATIONAL SEARCH REPORT

International Application No PCT/SU 88/00156

## I. CLASSIFICATION OF SUBJECT MATTER (if several classification symbols apply, indicate all) [6]

According to International Patent Classification (IPC) or to both National Classification and IPC

Int. Cl.[4]    C 07 K 1/04

## II. FIELDS SEARCHED

| Minimum Documentation Searched [7] | |
|---|---|
| Classification System | Classification Symbols |
| Int. Cl.[4] | C 07 K 1/04 |

| Documentation Searched other than Minimum Documentation to the Extent that such Documents are Included in the Fields Searched [8] |
|---|
|  |

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | D. Stuart et al. "Tverdofazny sintez peptidov", 1971, Mir (Moscou), see pages 82-93 | 1,2 |
| A | "Reaksia na polimernykh podlozhkakh v organichennom sinteze", ped red. P. Xodzha; D. Sherringtona, 1983, Mir (Moscou), see pages 454-456 | 1,2 |
| A | WO, A1, 86/03494, (LKB BIOCHROM LIMITED), 19 June 1986 (19.06.86), see page 3, lines 11-37, page 4, lines 1-10 | 1,2 |
| A | & GB,A0, 8431178, (23.01.85) GB,A1, 2169901, (23.07.86) AU,A1, 5205486, (01.07.86) EP,A1, 204801, (17.12.86) ------------ | |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 13 March 1989 (13.03.89) | 25 April 1989 (25.04.89) |
| International Searching Authority | Signature of Authorized Officer |
| ISA/SU | |

Form PCT/ISA/210 (second sheet) (January 1985)